# EUROPÄISCHE PATENTANMELDUNG

(11) **EP 4 386 360 A1**
(43) Veröffentlichungstag der Anmeldung: **19.06.2024**
(21) Anmeldenummer: 23215982.2
(22) Anmeldetag: 12.12.2023
(51) Int. Cl.: G01N 21/45, G01N 21/84, G01N 33/18, G01N 33/28, G01N 21/31

(54) **VERFAHREN UND VORRICHTUNG ZUR ÜBERWACHUNG EINER ANLAGE DER LUFT- UND KLIMATECHNIK**

(30) Priorität: 13.12.2022 DE 102022213580
(71) Anmelder: IT Inventor GmbH, 48465 Schüttorf / Samern (DE)
(72) Erfinder: Satar, Mehmet, 48531 Nordhorn (DE)
(74) Vertreter: Werner & ten Brink PAe PartGes mbB Bentheim

(57) **Zusammenfassung**

Die vorgeschlagene Neuerung ist ein Verfahren und eine Vorrichtung zur Überwachung einer Anlage (10) der Luft- und Klimatechnik, wobei aufgrund einer ersten Beleuchtung (40) und einer späteren, im Vergleich zu der ersten Beleuchtung (40) spektral unterschiedlichen zweiten Beleuchtung (42) mittels einer Sensorik (30) eine erste und eine zweite Messung (44, 46) einer Szenerie unterhalb der Anlage (10) erfolgt, wobei aufgrund der Messungen (44, 46) zum Beispiel zwei Emissionsspektren (44, 46) mit Spektrallinien (48) resultieren, wobei in den Emissionsspektren (44, 46) jeweils ein Spektrallinienpaar bei gleichen Wellenlängen betrachtet und deren Amplitudenverhältnis ermittelt wird und wobei die Amplitudenverhältnisse miteinander verglichen werden und in Abhängigkeit vom Ergebnis des Vergleichs ggf. eine Fehlerbehandlung (54) erfolgt.

## Beschreibung

Die hier vorgeschlagene Neuerung betrifft das technische Gebiet der Auffang- und Rückhaltesysteme für wassergefährdende Stoffe. Solche Auffang- und Rückhaltesysteme befinden sich zum Beispiel räumlich unterhalb einer im Folgenden kurz als Anlage bezeichneten Anlage der Luft- und Klimatechnik, zum Beispiel unterhalb eines Klimageräts oder einer Gruppe von Klimageräten. Zu solchen Anlagen gehören insbesondere Kälteanlagen, Klimaanlagen und Wärmepumpen, bei letzteren insbesondere die sogenannten Außengeräte. Ein solches Auffang- und Rückhaltesystem umfasst üblicherweise zumindest eine Auffangwanne oder diesem ist zumindest eine Auffangwanne zumindest zugeordnet, zum Beispiel indem das Auffang- und Rückhaltesystem in oder über der zumindest einen Auffangwanne platziert ist. Die Auffangwanne fängt im Fehlerfall aus der Anlage austretende Flüssigkeiten auf, zum Beispiel Glykol, Öl oder dergleichen (wassergefährdende Stoffe).

Vorrichtungen, mittels derer im Fehlerfall in der Auffangwanne befindliche, aus der Anlage ausgetretene Flüssigkeiten - im Folgenden einzeln und zusammen kurz als Fluid bzw. Fluide bezeichnet - in der Auffangwanne zurückgehalten oder detektiert werden, sind zum Beispiel aus der EP 2 335 794 B oder der EP 2 716 827 B bzw. der EP 3 301 440 B bekannt.

Eine Aufgabe der hier vorgeschlagenen Neuerung besteht darin, eine weitere Möglichkeit zur Detektion eines im Fehlerfall aus einer Anlage der eingangs genannten Art austretenden Fluids anzugeben.

Zur Lösung dieser Aufgabe sind ein Verfahren und eine nach dem Verfahren arbeitende Vorrichtung mit den Merkmalen des unabhängigen Verfahrensanspruchs bzw. des unabhängigen Vorrichtungsanspruchs vorgesehen.

Beispiele für im Fehlerfall aus einer Anlage der eingangs genannten Art austretende Fluide sind Glykol oder Öl. Der Begriff "Glykol" ist eine umgangssprachliche Bezeichnung für (Mono-) Ethylenglykol und Glykol wird in einer solchen Anlage bekanntlich als Frostschutzmittel eingesetzt. Im Falle einer Leckage solcher Anlagen droht ein Austritt des Frostschutzmittels. Weil Glykol als wassergefährdender Stoff eingestuft und überdies gesundheitsschädlich ist, muss bei einer Leckage speziell ein Versickern des Frostschutzmittels oder ein sonstiger Übergang des Frostschutzmittels in die Umwelt verhindert werden.

Das hier vorgeschlagene Verfahren und die hier vorgeschlagene, nach dem Verfahren arbeitende Vorrichtung sind jeweils zur Überwachung zumindest einer Anlage der eingangs genannten Art bestimmt und eingerichtet.

Das Verfahren (Überwachungsverfahren) zur Überwachung einer Anlage der eingangs genannten Art wird mittels einer dafür bestimmten und eingerichteten und im Folgenden oftmals kurz als Vorrichtung bezeichneten Überwachungsvorrichtung ausgeführt. Die Vorrichtung umfasst eine Sensorik mit zumindest einem optischen Sensor und eine Lichtquelle mit zumindest einem Leuchtmittel. Indem die Sensorik zumindest einen optischen Sensor umfasst, handelt es sich bei der Sensorik entsprechend um eine optische Sensorik. Die Sensorik und der oder jeder davon umfasste Sensor sowie die Lichtquelle und das oder jedes davon umfasste Leuchtmittel sind auf den Boden einer von der Anlage umfassten oder dieser zugeordneten Auffangwanne ausgerichtet oder allgemein in die Auffangwanne gerichtet. In der Auffangwanne kann sich Wasser befinden. Auf der Wasseroberfläche oder mit dem Wasser vermischt kann sich ein aus der Anlage ausgetretenes Fluid befinden. Mit der Ausrichtung von Sensorik und Lichtquelle auf den Boden der Auffangwanne beleuchtet die Lichtquelle im Falle von in der Auffangwanne befindlichem Wasser die Wasseroberfläche und nimmt die Sensorik zum Beispiel ein Bild der Wasseroberfläche auf.

Im Rahmen des Verfahrens erfolgt mittels der Lichtquelle, zum Beispiel mittels jeweils eines davon umfassten Leuchtmittels, eine erste Beleuchtung und - mit zeitlichem Abstand - eine im Vergleich zu der ersten Beleuchtung spektral unterschiedliche zweite Beleuchtung für eine mittels der Sensorik aufgenommene erste und zweite Messung. Die beiden Messungen entstehen dann ebenfalls mit einem entsprechenden zeitlichen Abstand. Im Falle von Wasser in der Auffangwanne erfolgen also eine erste und eine zweite Beleuchtung der Wasseroberfläche und bei der jeweiligen Beleuchtung erfolgt jeweils eine Messung, also eine erste und eine zweite Messung. Im Falle von einem aus der Anlage ausgetretenen Fluid wird/ist dieses von der Beleuchtung sowie der Messung stets ebenfalls erfasst. Vereinfacht ausgedrückt wird bei der ersten Beleuchtung die Wasseroberfläche zum Beispiel mit rotem Licht oder Licht in diesem Wellenlängenbereich und bei der zweiten Beleuchtung zum Beispiel mit blauem Licht oder Licht in diesem Wellenlängenbereich beleuchtet und es resultieren eine erste Messung mit der ersten Beleuchtung und eine zweite Messung mit der zweiten Beleuchtung.

Ein Erfassungsbereich der Sensorik, zum Beispiel des oder jedes davon umfassten Sensors, ist auf die Wellenlängenbereiche abgestimmt, mit denen die erste und zweite Beleuchtung erfolgen. Im Falle einer Sensorik mit Sensoren zum Beispiel in Form von Photodioden ist dies gewährleistet, indem der Erfassungsbereich eines ersten Sensors dem Wellenlängenbereich der ersten Beleuchtung entspricht oder den Wellenlängenbereich der ersten Beleuchtung zumindest umfasst und der Erfassungsbereich eines zweiten Sensors dem Wellenlängenbereich der zweiten Beleuchtung entspricht oder den Wellenlängenbereich der zweiten Beleuchtung zumindest umfasst. Im Falle einer Sensorik mit einem Sensor in Form eines CCD-Flächen- oder CCD-Zeilensensors ist dies gewährleistet, indem der Erfassungsbereich des Sensors den Wellenlängenbereich der ersten und zweiten Beleuchtung umfasst oder jeweils ein solcher Sensor den Wellenlängenbereich der ersten bzw. zweiten Beleuchtung umfasst.

Aufgrund der ersten und zweiten Messung resultieren jeweils ein erster Messwert und ein zweiter Messwert, zum Beispiel in Form eines Ausgangssignals jeweils eines von der Sensorik umfassten Sensors (zum Beispiel einer Photodiode). Die Messwerte werden im Folgenden als Datum bezeichnet: erster Messwert/erstes Datum, zweiter Messwert/zweites Datum. Im Rahmen einer Auswertung der Messungen wird ein Verhältnis des ersten und zweiten Datums jeder Messung gebildet. Es wird also ein Verhältnis des ersten und zweiten Datums der ersten Messung sowie ein Verhältnis des ersten und zweiten Datums der zweiten Messung gebildet. Im Rahmen einer von dem Verfahren umfassten Fallunterscheidung werden die ermittelten Verhältnisse miteinander verglichen. Die Verhältnisse können zum Beispiel gleich oder zumindest im Wesentlichen gleich sein. Dies ist ein Indiz dafür, dass sich in der Auffangwanne nur Wasser oder zumindest kein aus der Anlage ausgetretenes Fluid befindet. Wenn die Verhältnisse dagegen unterschiedlich sind, ist dies ein Indiz dafür, dass sich in der Auffangwanne auf der Wasseroberfläche ein Fluid befindet und das auf der Wasseroberfläche befindliche Fluid, zum Beispiel ein Ölfilm, mittels der beiden Messungen erfasst wurde. In Abhängigkeit vom Ergebnis des Vergleichs erfolgt schließlich im Rahmen des Verfahrens eine Fehlerbehandlung.

Der Vorteil des hier vorgeschlagenen Überwachungsverfahrens sowie einer nach dem Verfahren arbeitenden und insoweit Mittel zur Ausführung des Überwachungsverfahrens umfassenden Vorrichtung (Überwachungsvorrichtung) besteht insbesondere in der sicheren und berührungslosen Erkennung einer eventuellen Fehlersituation einer Anlage der eingangs genannten Art. Aufgrund der im Rahmen des Verfahrens erfolgenden Beleuchtung ist die Überwachung zudem unabhängig von wechselnden Lichtverhältnissen und es ist kein Eingriff in die jeweils überwachte Anlage notwendig.

Vorteilhafte Ausgestaltungen der hier vorgeschlagenen Neuerung sind Gegenstand der weiteren Ansprüche. Dabei verwendete Rückbeziehungen innerhalb der Ansprüche weisen auf die weitere Ausbildung des Gegenstandes des in Bezug genommenen Anspruchs durch die Merkmale des jeweiligen abhängigen Anspruchs hin. Sie sind nicht als ein Verzicht auf die Erzielung eines selbständigen, gegenständlichen Schutzes für die Merkmale oder Merkmalskombinationen eines abhängigen Anspruchs zu verstehen. Des Weiteren ist im Hinblick auf eine Auslegung der Ansprüche sowie der Beschreibung bei einer näheren Konkretisierung eines Merkmals in einem abhängigen Anspruch davon auszugehen, dass eine derartige Beschränkung in den jeweils vorangehenden Ansprüchen sowie einer allgemeineren Ausführungsform des vorgeschlagenen Verfahrens/der vorgeschlagenen Vorrichtung nicht vorhanden ist. Jede Bezugnahme in der Beschreibung auf Aspekte abhängiger Ansprüche ist demnach auch ohne speziellen Hinweis ausdrücklich als Beschreibung optionaler Merkmale zu lesen.

Des Weiteren sind die mit der Anmeldung eingereichten Ansprüche Formulierungsvorschläge ohne Präjudiz für die Erzielung weitergehenden Schutzes. Da speziell die Merkmale der abhängigen Ansprüche im Hinblick auf den Stand der Technik am Prioritätstag eigene und unabhängige Erfindungen bilden können, behält die Anmelderin sich vor, diese oder noch weitere, bisher nur in der Beschreibung und/oder Zeichnung offenbarte Merkmalskombinationen zum Gegenstand unabhängiger Ansprüche oder Teilungserklärungen zu machen. Sie können weiterhin auch selbständige Erfindungen enthalten, die eine von den Gegenständen der jeweils in Bezug genommenen Ansprüche unabhängige Gestaltung aufweisen.

Bei einer Ausführungsform des hier vorgeschlagenen Überwachungsverfahrens werden aufgrund der ersten und zweiten Beleuchtung resultierende Emissionsspektren betrachtet: Aufgrund der ersten und zweiten Beleuchtung resultiert ein mittels der ersten und zweiten Messung aufgenommenes erstes und zweites Emissionsspektrum mit Spektrallinien und in den beiden Emissionsspektren werden jeweils die Spektrallinien eines Spektrallinienpaars bei gleichen Wellenlängen betrachtet. Das aufgrund jeder Messung resultierende erste Datum und zweite Datum ist bei dieser Ausführungsform jeweils die Amplitude der betrachteten Spektrallinien.

Aufgrund der ersten und zweiten Beleuchtung und als Ergebnis der jeweils anschließenden ersten und zweiten Messung resultiert ein erstes und zweites Emissionsspektrum mit Spektrallinien, zum Beispiel im Wege einer im Rahmen des Verfahrens ausgeführten Spektralanalyse von unmittelbar mittels der Sensorik aufgenommenen Bilddaten. In den beiden Emissionsspektren wird jeweils ein Spektrallinienpaar betrachtet; Spektrallinienpaar im ersten Emissionsspektrum, Spektrallinienpaar im zweiten Emissionsspektrum. Zu jeder Spektrallinie gehört eine Wellenlänge und die Wellenlängen der beiden betrachteten Spektrallinienpaare sind gleich. Mit anderen Worten: Die Wellenlängen der ersten Spektrallinie des ersten und zweiten Spektrallinienpaars sind gleich und die Wellenlängen der zweiten Spektrallinie des ersten und zweiten Spektrallinienpaars sind gleich. Die beiden Emissionsspektren werden damit bei jeweils gleichen Wellenlängen betrachtet. Diese Betrachtung umfasst, dass im Rahmen des Verfahrens und einer von dem Verfahren umfassten Auswertung ein Verhältnis der Amplituden (Amplitudenverhältnis) der beiden Spektrallinienpaare gebildet wird. Mit anderen Worten: Es wird einerseits ein Verhältnis der Amplituden der ersten und zweiten Spektrallinie des ersten Emissionsspektrums sowie andererseits ein Verhältnis der Amplituden der ersten und zweiten Spektrallinie des zweiten Emissionsspektrums gebildet. Weiter im Rahmen des Verfahrens und einer von dem Verfahren umfassten Fallunterscheidung werden die ermittelten Amplitudenverhältnisse miteinander verglichen. Die Amplitudenverhältnisse können zum Beispiel gleich oder zumindest im Wesentlichen gleich sein. Dies ist ein Indiz dafür, dass sich in der Auffangwanne nur Wasser oder zumindest kein aus der Anlage ausgetretenes Fluid befindet. Wenn die Amplitudenverhältnisse unterschiedlich sind, ist dies ein Indiz dafür, dass sich in der Auffangwanne auf der Wasseroberfläche ein Fluid befindet und das auf der Wasseroberfläche befindliche Fluid, zum Beispiel ein Ölfilm, mittels der beiden Messungen erfasst wurde. In Abhängigkeit vom Ergebnis des Vergleichs erfolgt schließlich im Rahmen des Verfahrens eine Fehlerbehandlung.

Als Ergebnis einer Spektralanalyse wird auch ein sogenanntes Histogramm verstanden und soweit im Folgenden der Begriff Emissionsspektrum verwendet wird, umfasst dieser auch den Begriff Histogramm. Soweit von von einem Emissionsspektrum umfassten Spektrallinien gesprochen wird, handelt es sich im Falle eines Histogramms um entsprechende Abschnitte des Histogramms, nämlich die Position des Histogramms, welche einen jeweiligen Farbwert kodiert. Im Sinne einer durchgängigen Terminologie der hier vorgelegten Beschreibung umfasst damit nicht nur der Begriff Emissionsspektrum auch den Begriff Histogramm, sondern auch der Begriff Spektrallinie einen linienförmigen Abschnitt des Histogramms, also die Position des Histogramms, welche einen jeweiligen Farbwert kodiert.

Die oben genannte Aufgabe wird auch mittels einer Vorrichtung (Überwachungsvorrichtung) zur Überwachung einer Anlage der eingangs genannten Art gelöst, welche nach einem Verfahren wie hier und im Folgenden beschrieben arbeitet und dazu Mittel zur Durchführung des Verfahrens umfasst. Das Verfahren ist zur automatischen Ausführung bevorzugt in Form eines Steuerungsprogramms realisiert (das Steuerungsprogramm ist eine Implementierung des Verfahrens zur Überwachung einer Anlage der eingangs genannten Art in Soft- und/oder Firmware). Die Erfindung ist damit einerseits auch ein Steuerungsprogramm mit durch einen Computer ausführbaren Programmcodeanweisungen und andererseits ein Speichermedium mit einem derartigen Steuerungsprogramm, also ein Computerprogrammprodukt mit Programmcodemitteln, sowie schließlich auch eine Vorrichtung (Überwachungsvorrichtung), in deren Speicher als Mittel zur Durchführung des Verfahrens und seiner Ausgestaltungen ein solches Steuerungsprogramm geladen oder ladbar ist.

Wenn in der hier vorgelegten Beschreibung Verfahrensschritte oder Verfahrensschrittfolgen beschrieben werden, bezieht sich dies auf Aktionen, die aufgrund des Steuerungsprogramms oder unter Kontrolle des Steuerungsprogramms erfolgen, sofern nicht ausdrücklich darauf hingewiesen ist, dass einzelne Aktionen durch einen Benutzer veranlasst werden. Zumindest bedeutet jede Verwendung des Begriffs "automatisch", dass die betreffende Aktion aufgrund des Steuerungsprogramms oder unter Kontrolle des Steuerungsprogramms erfolgt.

Anstelle eines Steuerungsprogramms mit einzelnen Programmcodeanweisungen kann die Implementierung des hier und im Folgenden beschriebenen Verfahrens auch in Form von Firmware erfolgen. Dem Fachmann ist klar, dass anstelle einer Implementation in Software stets auch eine Implementation in Firmware oder in Firm- und Software oder in Firm- und Hardware möglich ist. Daher soll für die hier vorgelegte Beschreibung gelten, dass von dem Begriff Software oder dem Begriff Steuerungsprogramm auch andere Implementationsmöglichkeiten, nämlich insbesondere eine Implementation in Firmware oder in Firm- und Software oder in Firm- und Hardware, umfasst sind.

Die hier vorgeschlagene Neuerung ist insgesamt auch die Verwendung einer Vorrichtung oder eines Verfahrens wie hier und im Folgenden beschrieben zur automatischen Überwachung einer Anlage der eingangs genannten Art, zum Beispiel eine automatische Überwachung einer in einer Auffangwanne platzierten Anlage, insbesondere einer Anlage der Luft- und Klimatechnik, in Bezug auf einen Austritt eines wasser- und/oder umweltgefährdenden Fluids aus der Anlage.

Nachfolgend werden Ausführungsbeispiele der Neuerung anhand der Zeichnung näher erläutert. Einander entsprechende Gegenstände oder Elemente sind in allen Figuren mit den gleichen Bezugszeichen versehen. Die Ausführungsbeispiele sind nicht als Einschränkung der Neuerung zu verstehen. Vielmehr sind im Rahmen der vorliegenden Offenbarung durchaus auch Ergänzungen und Modifikationen möglich, insbesondere solche, die zum Beispiel durch Kombination oder Abwandlung von einzelnen in Verbindung mit den im allgemeinen oder speziellen Beschreibungsteil beschriebenen sowie in den Ansprüchen und/oder der Zeichnung enthaltenen Merkmalen oder Verfahrensschritten für den Fachmann im Hinblick auf die Lösung der Aufgabe entnehmbar sind und durch kombinierbare Merkmale zu einem neuen Gegenstand oder zu neuen Verfahrensschritten bzw. Verfahrensschrittfolgen führen.

Es zeigen
- Fig. 1: eine Anlage der Luft- und Klimatechnik in Form eines Klimageräts mit einer Vorrichtung zu deren Überwachung,
- Fig. 2: ein Ablaufdiagramm zu dem hier vorgeschlagenen Verfahren zur Überwachung einer Anlage der Luft- und Klimatechnik,
- Fig. 3: zwei Emissionsspektren,
- Fig. 4: die Anlage gemäß Fig. 1 und ein im Fehlerfall aus der Anlage austretendes Fluid,
- Fig. 5: zwei (weitere) Emissionsspektren,
- Fig. 6: die Vorrichtung aus Fig. 1 mit weiteren Einzelheiten und
- Fig. 7: ein Beispiel für ein mittels der Vorrichtung aufgenommenes Digitalbild.

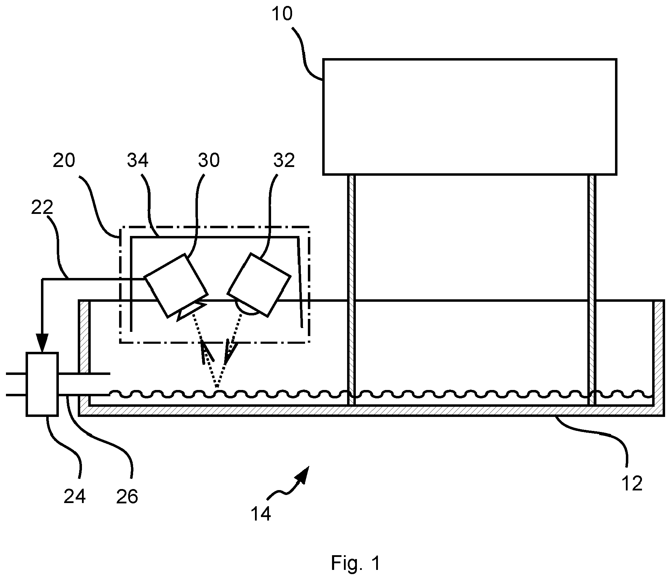

Die Darstellung in Figur 1 zeigt in schematisch stark vereinfachter Form als Beispiel für eine Anlage 10 der Luft- und Klimatechnik ein Klimagerät 10. Dieses ist in einer Auffangwanne 12 eines im Folgenden kurz als Rückhaltesystem 14 bezeichneten Auffang- und Rückhaltesystems 14 platziert.

Gegenstand der hier vorgeschlagenen Neuerung ist eine automatische Überwachung einer solchen oder ähnlichen Anlage 10. Die Überwachung erfolgt mittels einer im Folgenden kurz als Vorrichtung 20 bezeichneten Überwachungsvorrichtung 20 sowie mittels eines Verfahrens (Überwachungsverfahren) zur Überwachung der jeweiligen Anlage 10 unter Verwendung der Vorrichtung 20.

Im Fehlerfall wird mittels der Vorrichtung 20 ein Fehlersignal 22 erzeugt; mittels der Vorrichtung 20 ist im Fehlerfall ein Fehlersignal 22 generierbar. Mittels des Fehlersignals 22 wird zum Beispiel eine der Auffangwanne 12 zugeordnete Absperreinrichtung angesteuert; das Fehlersignal 22 oder ein aufgrund des Fehlersignals 22 resultierendes Ansteuersignal wird der Absperreinrichtung zugeführt/die Absperreinrichtung wird mit dem Fehlersignal 22 oder einem aufgrund des Fehlersignals 22 resultierenden Ansteuersignal beaufschlagt. Bei der Absperreinrichtung handelt es sich zum Beispiel um ein Ventil (Absperrventil) 24 stromabwärts eines Ablaufs 26 aus der Auffangwanne 12 (oder eine Mehrzahl solcher Ventile 24 im Falle einer Mehrzahl von Abläufen 26).

Die Vorrichtung 20 umfasst eine Sensorik 30 mit zumindest einem optischen Sensor. Als Sensor fungiert zum Beispiel ein Sensor aus der folgenden Gruppe: lichtempfindlicher Flächensensor, wie er zum Beispiel von Digitalkameras bekannt ist, insbesondere CCD-Flächensensor (CCD-Sensor), lichtempfindlicher Zeilensensor, insbesondere CCD-Zeilensensor, Indiumgalliumarsenid-Infrarotdetektor (InGaAs-Detektor).

Die Vorrichtung 20 umfasst des Weiteren eine Lichtquelle 32 mit zumindest einem Leuchtmittel, also zum Beispiel eine Lichtquelle 32 mit genau einem Leuchtmittel oder eine Lichtquelle 32 mit genau zwei Leuchtmitteln oder eine Lichtquelle 32 mit zwei oder mehr Leuchtmitteln, insbesondere eine Lichtquelle 32 mit einer der Anzahl der im Rahmen des Verfahrens jeweils durchgeführten Messungen 44, 46 entsprechenden Anzahl von Leuchtmitteln. Die Vorrichtung 20 umfasst schließlich auch ein Gehäuse 34. Das Gehäuse 34 nimmt die Sensorik 30 und die Lichtquelle 32 auf. Das Gehäuse 34 fungiert als Licht abschirmende Kammer und reduziert den Einfluss von Fremdlicht. Das Gehäuse 34 ist in Richtung auf die Auffangwanne 12 offen oder zumindest teilweise offen und erlaubt zumindest den Austritt von mittels der Lichtquelle 32 erzeugtem Licht und den Eintritt reflektierten Lichts in Richtung auf die Sensorik 30.

Die Lichtquelle 32, nämlich das oder jedes davon umfasste Leuchtmittel, strahlt bevorzugt unter einem Winkel ungleich 90° in die Auffangwanne 12. Wenn sich in der Auffangwanne 12 Wasser befindet, strahlt die Lichtquelle 32 entsprechend unter diesem Winkel auf die Wasseroberfläche oder ein sich ggf. auf der Wasseroberfläche befindendes Fluid (Öl oder Glykol). Sensorik 30 und Lichtquelle 32 müssen nicht notwendig - wie schematisch vereinfacht gezeigt - räumlich voneinander getrennt sein. Als Lichtquelle 32 kann zum Beispiel ein Ringlicht fungieren, das aus der Richtung der Sensorik 30 in die Auffangwanne 12 strahlt. Ein Ringlicht als Lichtquelle 32 umgibt die Sensorik 30 kreisförmig oder zumindest abschnittsweise entlang einer Kreislinie.

Eine Messung mittels der Vorrichtung 20 erfolgt unter zumindest zwei Wellenlängen oder Wellenlängenbereichen (entsprechend: Frequenzen oder Frequenzbereichen). D. h. die Lichtquelle 32, zum Beispiel jeweils ein von der Lichtquelle 32 umfasstes Leuchtmittel, strahlt zunächst Licht einer ersten Wellenlänge oder eines ersten Wellenlängenbereichs - erste Beleuchtung 40 mit bevorzugt kohärentem Licht - und sodann in einem vorgegebenen oder vorgebbaren zeitlichen Abstand Licht einer zweiten Wellenlänge oder eines zweiten Wellenlängenbereichs - spektral unterschiedliche zweite Beleuchtung 42 mit bevorzugt kohärentem Licht - aus. Mittels der Sensorik 30 wird jeweils die aufgrund des ausgestrahlten Lichts resultierende Reflexion gemessen. Bei der ersten Beleuchtung 40 oder auf die erste Beleuchtung 40 erfolgt bzw. folgt also eine erste Messung 44 und bei der zweiten Beleuchtung 42 oder auf die zweite Beleuchtung 42 erfolgt bzw. folgt entsprechend eine zweite Messung 46. Es resultieren also Messungen 44, 46 mit einem zeitlichen Abstand, der dem zeitlichen Abstand oder im Wesentlichen dem zeitlichen Abstand der Beleuchtungen 40, 42 entspricht.

Auch wenn die erste Beleuchtung 40 und die zweite Beleuchtung 42 in der vorangehenden Beschreibung als Beleuchtung mit Licht, also entsprechend dem allgemeinen Sprachverständnis als Beleuchtung mit Licht im sichtbaren Bereich, beschrieben wurde, soll als "Beleuchtung mit Licht" ganz allgemein eine Bestrahlung mit elektromagnetischer Strahlung gelten und die Lichtquelle 32, nämlich das oder jedes davon umfasste Leuchtmittel, ist dann allgemein ein elektromagnetischer Strahler. Im Interesse einer besseren Lesbarkeit der hier vorgelegten Beschreibung basiert diese auf einer Beleuchtung 40, 42 mit sichtbarem Licht, denn dann sind gebräuchliche und kurze Bezeichnungen verwendbar. Trotzdem sind stets auch allgemein eine Bestrahlung mit elektromagnetischer Strahlung ohne Beschränkung auf den sichtbaren Bereich sowie eine entsprechende Sensorik 30 und eine entsprechende Strahlquelle 32 mitzulesen.

Als Sensorik 30 fungiert zum Beispiel - wie für das hier erläuterte Ausführungsbeispiel in den Figuren gezeigt - zumindest ein Sensor, wie er von Digitalkameras oder Zeilenkameras bekannt ist. Als Sensorik 30 fungieren alternativ zum Beispiel zumindest zwei in unterschiedlichen Spektralbereichen sensitive Photodioden.

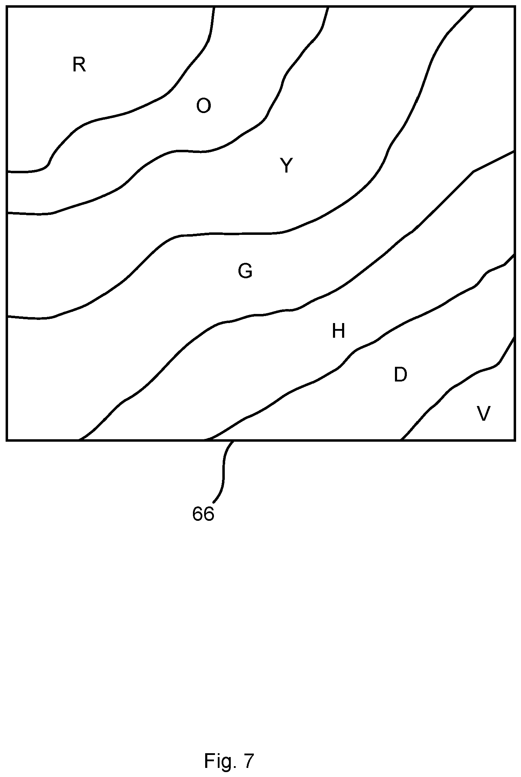

Der Begriff Messung 44, 46 meint im Zusammenhang mit der hier vorgelegten Beschreibung einerseits das Datum oder die Datenmenge, das bzw. die mittels der Sensorik 30 aufgrund der Reflexion der jeweiligen Beleuchtung 40, 42 aufnehmbar ist und im Rahmen des Verfahrens aufgenommen wird, sowie andererseits auch den Vorgang zum Erhalt eines solches Datums bzw. einer solchen Datenmenge. Das Ergebnis der Messung 44, 46 kann eine Datenmenge sein, nämlich ein Digitalbild 66 (Fig. 7), das in an sich bekannter Art und Weise in Zeilen und Spalten angeordnete sogenannte Pixel umfasst, nämlich im Falle einer einen CCD-(Flächen-)Sensor umfassenden Sensorik 30. Das Ergebnis der Messung 44, 46 kann auch ein Digitalbild 66 sein, das eine Mehrzahl von in einer Zeile angeordnete Pixel umfasst, nämlich im Falle einer einen CCD-Zeilensensor umfassenden Sensorik 30. Im Falle einer Photodioden umfassenden Sensorik 30 ist das Ergebnis der Messung 44, 46 jeweils ein aufgrund eines Ausgangsignals jeder Photodiode resultierendes Datum.

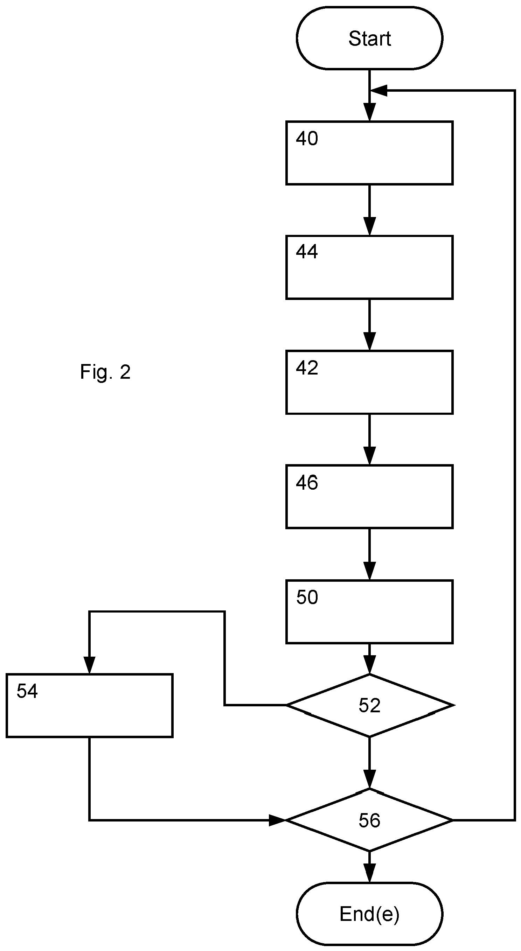

Die Darstellung in Figur 2 zeigt den vorstehend beschriebenen Ablauf schematisch vereinfacht in Form eines Ablaufdiagramms und das Ablaufdiagramm ist eine grafische Darstellung des hier vorgeschlagenen Verfahrens zur automatischen Überwachung einer jeweiligen Anlage 10, insbesondere einer Anlage 10 der Luft- und Klimatechnik. Anstelle zweier Beleuchtungen 40, 42 und entsprechend zweier Messungen 44, 46 sind auch mehr als zwei Beleuchtungen 40, 42 und eine entsprechende Anzahl von Messungen 44, 46 möglich.

An die letzte Messung 46 schließt eine Auswertung 50 der zumindest zwei Messungen 44, 46 an. Im Rahmen der Auswertung 50 werden die Messungen 44, 46 bewertet. Die Auswertung 50 wird weiter unten mit weiteren Details beschrieben. An die Auswertung 50 schließt eine Fallunterscheidung 52 an. Diese bewirkt eine vom Ergebnis der Auswertung 50 abhängige Verzweigung im Rahmen des Ablaufs. Wenn die Fallunterscheidung 52 das Vorliegen einer Fehlersituation ergibt, wird zu einer Fehlerbehandlung 54 verzweigt und dort wird das Fehlersignal 22 erzeugt. Ansonsten wird der Ablauf fortgesetzt und bis zur Erfüllung eines Abbruchkriteriums 56 wird der Prozess des Beleuchtens 40, 42 (erste Beleuchtung 40, zweite Beleuchtung 42), des Messens 44, 46 (erste Messung 44, zweite Messung 46) und des Auswertens 50 (Auswertung 50) mit anschließender Fallunterscheidung 52 zyklisch fortgesetzt. Ein jeder Zyklus umfasst also das Beleuchten 40, 42, das Messen 44, 46 und das Auswerten 50.

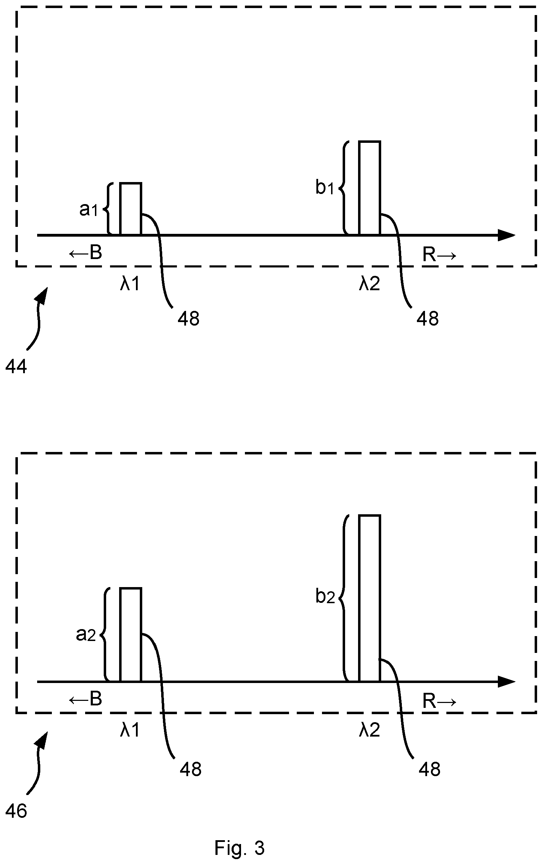

Die Darstellung in Figur 3 zeigt das Ergebnis einer Verarbeitung zweier möglicher Messungen 44, 46. Im Falle von mittels der Sensorik 30 als Messung 44, 46 aufgenommenen Digitalbildern 66 umfasst die Verarbeitung eine Spektralanalyse der Digitalbilder 66, also eine Spektralanalyse jeder Messung 44, 46. Das Ergebnis der Verarbeitung der Messungen 44, 46 ist jeweils ein Emissionsspektrum 44, 46 (erstes Emissionsspektrum 44, zweites Emissionsspektrum 46). Das erste Emissionsspektrum 44 (Figur 3: oben) resultiert aufgrund der ersten Messung 44; das zweite Emissionsspektrum 46 (Figur 3: unten) resultiert aufgrund der zweiten Messung 46. Für das Ergebnis der Verarbeitung der Messungen 44, 46, nämlich das jeweils resultierende Emissionsspektrum 44, 46, und die zugrunde liegenden Messungen 44, 46 werden dieselben Bezugsziffern verwendet.

Die Darstellung in Figur 3 zeigt die Emissionsspektren 44, 46 über den Wellenlängen des sichtbaren Lichts (veranschaulicht durch die Beschriftung mit B links und R rechts - für blaues Licht bzw. rotes Licht) und der mittels der Sensorik 30 erfasste Wellenlängenbereich wird mittels der horizontalen Koordinatenachse veranschaulicht. Je nach spektraler Empfindlichkeit der Sensorik 30 und des oder jedes davon umfassten Sensors ergeben sich ggf. Emissionsspektren 44, 46 in anderen Wellenlängenbereichen, also zum Beispiel im ultravioletten Bereich oder auch im ultravioletten Bereich oder im Infrarotbereich oder auch im Infrarotbereich.

Die Emissionsspektren 44, 46 zeigen aufgrund der ersten Beleuchtung 40 bzw. der zweiten Beleuchtung 42 unterschiedliche Spektrallinien 48 jeweils unterschiedlicher Höhe a, b (zur Unterscheidung indiziert: a₁, b₁; a₂, b₂).

Es ist darauf hinzuweisen, dass die aufgrund der Messungen 44, 46 resultierenden Emissionsspektren 44, 46 nicht auf die gezeigten zwei Spektrallinien 48 beschränkt sein müssen, sondern weitere (im Zuge der Auswertung 50 nicht betrachtete und hier nicht gezeigte) Spektrallinien 48 umfassen können.

In der Darstellung in Figur 3 sind die Spektrallinien bei symbolisch mit λ1 und λ2 bezeichneten Wellenlängen oder Wellenlängenbereichen (entsprechend: Frequenzen oder Frequenzbereichen f1 ~ 1/λ1, f2 ~ 1/λ2) schematisch vereinfacht als Säulen gezeigt. Die Spektrallinie 48 mit der Amplitude a₁ im ersten Emissionsspektrum 44 gehört zu der Wellenlänge/dem Wellenlängenbereich λ1, die Spektrallinie 48 mit der Amplitude b₁ im ersten Emissionsspektrum 44 gehört zu der Wellenlänge/dem Wellenlängenbereich λ2, die Spektrallinie 48 mit der Amplitude a₂ im zweiten Emissionsspektrum 46 gehört zu der Wellenlänge/dem Wellenlängenbereich λ1 und die Spektrallinie 48 mit der Amplitude b₂ im zweiten Emissionsspektrum 46 gehört zu der Wellenlänge/dem Wellenlängenbereich λ2.

Im Zuge des hier vorgeschlagenen Verfahrens werden jeweils zwei Spektrallinien 48 betrachtet. Jeweils zwei Spektrallinien 48 werden zusammen als Spektrallinienpaar bezeichnet und bilden zusammen ein Spektrallinienpaar. In den beiden Emissionsspektren 44, 46 wird bei jeweils gleichen Wellenlängen oder Wellenlängenbereichen λ1, λ2 jeweils ein Spektrallinienpaar betrachtet. Das im ersten Emissionsspektrum 44 betrachtete Spektrallinienpaar und das im zweiten Emissionsspektrum 46 betrachtete Spektrallinienpaar werden zur Unterscheidung als erstes Spektrallinienpaar bzw. zweites Spektrallinienpaar bezeichnet; das erste Spektrallinienpaar bei λ1, λ2 gehört zum ersten Emissionsspektrum 44 und das zweite Spektrallinienpaar bei ebenfalls λ1, λ2 gehört zum zweiten Emissionsspektrum 46.

Im Rahmen des Verfahrens und der davon umfassten Auswertung 50 wird ein Verhältnis (Quotient) der Amplituden a₁, b₁; a₂, b₂ der beiden Spektrallinienpaare gebildet. Das Verhältnis der Amplituden ist dabei das Verhältnis der Amplituden der von jeweils einem Spektrallinienpaar umfassten Spektrallinien 48. Es ergibt sich also ein erstes Amplitudenverhältnis (a₁/b₁) für das erste Spektrallinienpaar und ein zweites Amplitudenverhältnis (a₂/b₂) für das zweite Spektrallinienpaar.

Bei der in Figur 3 gezeigten Situation entspricht das Amplitudenverhältnis des ersten Spektrallinienpaars im aufgrund der ersten Beleuchtung 40 resultierenden ersten Emissionsspektrum 44 dem Amplitudenverhältnis des zweiten Spektrallinienpaars im aufgrund der zweiten Beleuchtung 42 resultierenden zweiten Emissionsspektrum 46: a₁/b₁ = a₂/b_{2.}

Eine solche Gleichheit oder zumindest annähernde Gleichheit (zum Beispiel mit einer Abweichung von ± 10%) bedeutet im Rahmen des hier vorgeschlagenen Überwachungsverfahrens, dass die Wasseroberfläche in der Auffangwanne 12 nicht verschmutzt ist, sich also auf der Wasseroberfläche kein im Rahmen des hier vorgeschlagenen Verfahrens zu erkennendes Fluid befindet.

Der Vergleich der Amplitudenverhältnisse (a₁/b₁, a₂/b₂) erfolgt zum Beispiel im Rahmen der Fallunterscheidung 52. Die Fallunterscheidung 52 kann als Prüfung auf Gleichheit oder zumindest annähernde Gleichheit der Amplitudenverhältnisse implementiert sein. Als Ergebnis der Fallunterscheidung 52 wird zur Fehlerbehandlung 54 verzweigt, wenn die Amplitudenverhältnisse im oben geschriebenen Sinne nicht gleich oder nicht zumindest annähernd gleich sind. Andernfalls wird der Ablauf ohne eine Verzweigung zur Fehlerbehandlung 54 fortgesetzt.

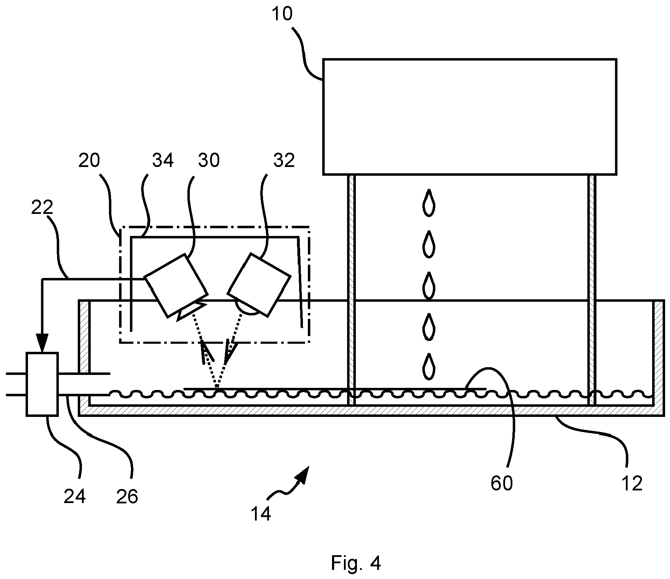

Die Darstellung in Figur 4 zeigt nun - auf der Basis der Darstellung in Figur 1 - eine Fehlersituation. Dies wird zum einen mittels der von der Anlage 10 ausgehenden Tropfenlinie gezeigt. Diese stellt ein aus der Anlage 10 im Fehlerfall austretendes Fluid dar, zum Beispiel Öl. Zum anderen wird dies mittels einer dünnen Schicht auf der Oberfläche des in der Auffangwanne 12 befindlichen Wassers gezeigt. Bei austretendem Öl handelt es sich bei dieser dünnen Schicht entsprechend um einen Ölfilm 60.

Im Falle eines Ölfilms 60 auf einer Wasseroberfläche sind die resultierenden sichtbaren Interferenzfarben ("Regenbogenfarben") an sich bekannt. Diese sind das Ergebnis einer Überlagerung einerseits an der Oberfläche des Ölfilms 60 und andererseits an der darunterliegenden Wasseroberfläche reflektierter Lichtwellen sowie einer resultierenden, zum Teil konstruktiven und zum Teil destruktiven Interferenz. Dies und die zugrunde liegenden physikalischen Gesetzmäßigkeiten sind an sich bekannt. Für andere dünne Schichten auf einer Wasseroberfläche gilt dies entsprechend.

Im Rahmen des hier vorgeschlagenen Verfahrens zur Überwachung einer jeweiligen Anlage 10, insbesondere einer Anlage 10 der Luft- und Klimatechnik, mittels einer Vorrichtung 20 wird - kurz gefasst - automatisch ermittelt, ob sich auf der Wasseroberfläche in der Auffangwanne 12 ein Ölfilm 60 oder allgemein eine Interferenzfarben erzeugende dünne Schicht eines Fluids befindet. Dies gilt für eine Beleuchtung 40, 42 mit sichtbarem Licht. Im Falle einer Beleuchtung 40, 42 im Infrarotbereich wird automatisch ermittelt, ob sich auf der Wasseroberfläche in der Auffangwanne 12 eine dünne Schicht mit einer im Vergleich zu dem Wasser in der Auffangwanne 12 anderen Wärmekapazität befindet.

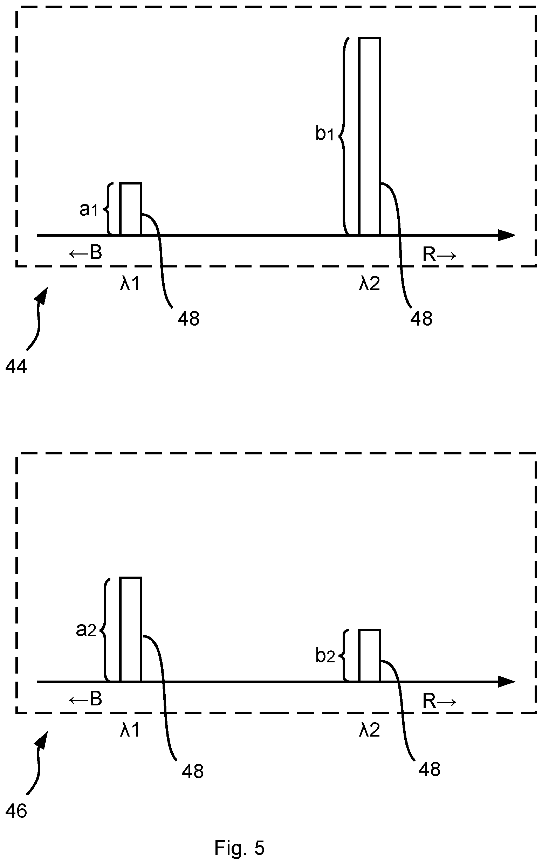

Bei der kontinuierlich zyklischen Beleuchtung 40, 42 der Wasseroberfläche und der Aufnahme von Messungen 44, 46 bezüglich der Wasseroberfläche in der Auffangwanne 12 wird im Falle einer Fehlersituation gemäß Figur 4 der Ölfilm 60 (oder die jeweilige dünne Schicht) beleuchtet 40, 42 und gemessen 44, 46. Die Darstellung in Figur 5 zeigt - analog zu der Darstellung in Figur 3 - das Ergebnis einer Verarbeitung zweier möglicher Messungen 44, 46 im Falle eines Ölfilms 60 auf der Wasseroberfläche. Die Verarbeitung ist dieselbe Verarbeitung (die Verarbeitung erfolgt weiterhin im Rahmen des Verfahrens gemäß Figur 2) wie zuvor beschrieben und das Ergebnis der Verarbeitung ist jeweils ein Emissionsspektrum 44, 46 mit Spektrallinien 48 und Spektrallinienpaaren.

Erneut (wie bei der in Figur 3 gezeigten Situation) zeigen die Emissionsspektren 44, 46 aufgrund der ersten Beleuchtung 40 bzw. der zweiten Beleuchtung 42 (jeweils bevorzugt mit kohärentem Licht oder jedenfalls Licht eines begrenzten Wellenlängenbereichs) unterschiedliche und jeweils als Säulen gezeigte Spektrallinien 48 jeweils unterschiedlicher Höhe a₁, b₁; a₂, b₂. Auch hier gehört die Spektrallinie 48 mit der Amplitude a₁ im ersten Emissionsspektrum 44 zu der Wellenlänge/dem Wellenlängenbereich λ1, die Spektrallinie 48 mit der Amplitude b₁ im ersten Emissionsspektrum 44 gehört zu der Wellenlänge/dem Wellenlängenbereich λ2, die Spektrallinie 48 mit der Amplitude a₂ im zweiten Emissionsspektrum 46 gehört zu der Wellenlänge/dem Wellenlängenbereich λ1 und die Spektrallinie 48 mit der Amplitude b₂ im zweiten Emissionsspektrum 46 gehört zu der Wellenlänge/dem Wellenlängenbereich λ2. Wesentlich ist, dass hier nun das Amplitudenverhältnis der betrachteten Spektrallinien 48 (Spektrallinienpaare) ungleich ist: a₁/b₁ ≠ a₂/b₂. Dies ist ein Indiz dafür, dass sich ein dünner Film, nämlich ein bei einer Beleuchtung 40, 42 mit sichtbarem Licht Interferenzfarben erzeugender dünner Film, zum Beispiel ein Ölfilm 60, auf der Oberfläche des Wassers in der Auffangwanne 12 befindet. In diesem Fall wird also durch die Auswertung 50 der Messungen 44, 46 und die anschließende Fallunterscheidung 52 eine Fehlersituation erkannt und durch eine solche Auswertung 50 der Messungen 44, 46, nämlich der jeweiligen Emissionsspektren 44, 46, und die anschließende Fallunterscheidung 52 ist eine Fehlersituation automatisch erkennbar, wobei die Auswertung 50 der Messungen 44, 46 einen Vergleich der Spektrallinien 48 der bei spektral unterschiedlicher, bevorzugt kohärenter Beleuchtung 40, 42 jeweils resultierenden Emissionsspektren 44, 46 umfasst.

Auch hier erfolgt der Vergleich der beiden Quotienten (a₁/b₁, a₂/b₂) zum Beispiel im Rahmen der Fallunterscheidung 52. Als Ergebnis der Fallunterscheidung 52 wird zur Fehlerbehandlung 54 verzweigt, wenn die beiden Quotienten (Amplitudenverhältnisse) im weiter oben geschriebenen Sinne nicht gleich oder nicht zumindest annähernd gleich sind. Andernfalls wird der Ablauf ohne eine Verzweigung zur Fehlerbehandlung 54 fortgesetzt.

Im Falle einer Photodioden umfassenden Sensorik 30 (nicht gezeigt) entfällt die Notwendigkeit einer separaten Spektralanalyse, denn jede Photodiode liefert aufgrund einer entsprechenden spektralen Sensitivität als Ausgangssignal bereits ein Maß für die oben beschriebenen Amplituden a₁, b₁, a₂, b₂. Hier sind direkt die oben beschriebene Ermittlung der Amplitudenverhältnisse und der Vergleich der Amplitudenverhältnisse möglich.

Im Falle einer Beleuchtung 40, 42 im Infrarotbereich gilt das oben Geschriebene entsprechend. Anstelle der bei einer Beleuchtung 40, 42 mit sichtbarem Licht im Fehlerfall resultierenden Interferenzfarben ist oder umfasst die Messung 44, 46, insbesondere die zweite Messung 46, dann aufgrund der eingestrahlten Wärmeenergie ein Wärmebild.

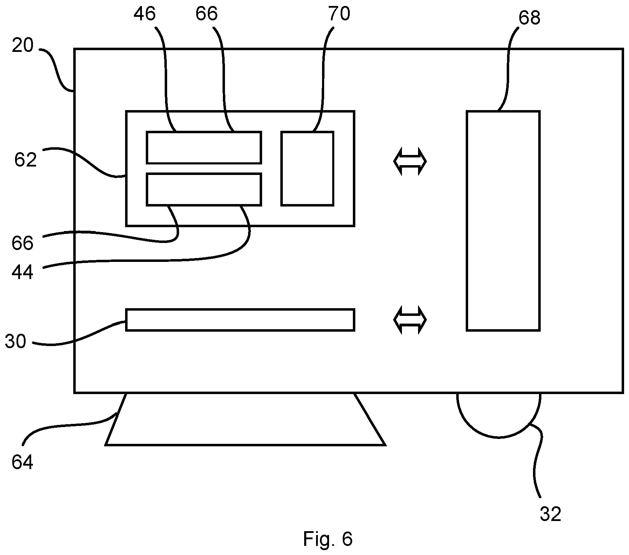

Die Darstellung in Figur 6 zeigt die Vorrichtung 20 mit weiteren Details, aber schematisch stark vereinfacht sowie - beispielhaft - ohne die in Figur 1 (dort ebenso nur beispielhaft) gezeigte räumliche Trennung von Sensorik 30 und Lichtquelle 32. Die Vorrichtung 20 umfasst die Sensorik 30, einen Speicher 62 und je nach Sensorik 30 eine Optik 64. Mittels der Optik 64 erfolgt in grundsätzlich an sich bekannter Art und Weise eine Abbildung einer von der Optik 64 erfassten Szenerie auf die Oberfläche der Sensorik 30. Die Sensorik 30 oder die Sensorik 30 und die Optik 64 erfassen einen Bereich der Wasseroberfläche in der Auffangwanne 12. Ein mittels der Sensorik 30 aufgenommenes Datum oder mittels der Sensorik 30 aufgenommene Daten, insbesondere ein Digitalbild 66 - im Folgenden einzeln und zusammenfassend kurz als Digitalbild 66 bezeichnet -, wird in den Speicher 62 geladen. Das/jedes Digitalbild 66 ist die Messung 44, 46 oder das Ergebnis einer Messung 44, 46, nämlich einer Messung 44, 46 im Zuge des Verfahrens gemäß Figur 2.

Als Speicher 62 kann ein von der Sensorik 30 unabhängiger Speicher 62, ein von der Sensorik 30 umfasster Speicher 62 oder die Sensorik 30 selbst fungieren. Neben den bereits erwähnten Komponenten Sensorik 30, Speicher (Bildspeicher) 62 und Optik 64 umfasst die Vorrichtung 20 eine Verarbeitungseinheit 68 in Form von oder nach Art eines Mikroprozessors oder eines digitalen Signalprozessors. Diese Komponenten (Sensorik 30, Speicher 62, Optik 64, Verarbeitungseinheit 68) sind für sich genommen und in ihrer Funktion einzeln und in Kombination an sich bekannt, so dass eine weitergehende Erläuterung hier entbehrlich ist.

In den Speicher 62 ist ein Steuerungsprogramm 70 geladen. Das Steuerungsprogramm 70 ist eine Implementation des hier vorgeschlagenen Überwachungsverfahrens, also insbesondere eine Implementation des in der Darstellung in Figur 2 gezeigten Ablaufs und der von dem Ablauf umfassten Verfahrensschritte. Das Steuerungsprogramm 70 wird beim Betrieb der Vorrichtung 20 mittels deren Verarbeitungseinheit 68 ausgeführt. Das Steuerungsprogramm 70 umfasst Befehle, die bei dessen Ausführung mittels der Verarbeitungseinheit 68 die Vorrichtung 20 veranlassen, das hier vorgeschlagene Überwachungsverfahren auszuführen.

Mit dem in den Speicher 62 ladbaren/geladenen Steuerungsprogramm 70 umfasst die Vorrichtung 20 eine Implementation des hier vorgeschlagenen Überwachungsverfahrens. Mit anderen Worten umfasst die Vorrichtung 20 Mittel zur Ausführung des hier vorgeschlagenen Überwachungsverfahrens. Zu diesen Mitteln gehören insbesondere der Speicher 62, in den das Steuerungsprogramm 70 ladbar ist und zum Betrieb der Vorrichtung 20 geladen ist, das Steuerungsprogramm 70 und die Verarbeitungseinheit 68. Im weiteren Sinne gehören zu diesen Mitteln auch die Sensorik 30 und der oder jeder davon umfasste Sensor, eine eventuelle Optik 64 und die Lichtquelle 32.

Als Digitalbild 66 wird ein Bereich im Speicher 62 bezeichnet, der eine computerlesbare Form der mittels der Optik 64 und der Sensorik 30 aufgenommenen Verhältnisse im Erfassungsbereich der Optik 64 umfasst. Das Ergebnis der Messungen 44, 46 sind zum Beispiel Digitalbilder 66. Jedes davon umfasste Pixel repräsentiert einen Ort des aufgenommenen Bildes und kodiert einen Farb- oder Grauwert an diesem Ort (oder im Falle eines Infrarotbilds eine Temperatur). Die Emissionsspektren 44, 46 basieren zum Beispiel auf jeweiligen Digitalbildern 66 und sind zum Beispiel das Ergebnis einer Spektralanalyse eines jeweiligen Digitalbilds 66. In den Emissionsspektren 44, 46 werden - wie weiter oben beschrieben - die jeweiligen Spektrallinienpaare und deren Amplitudenverhältnisse betrachtet.

Die Darstellung in Figur 7 zeigt - in schematisch vereinfachter Form - ein Beispiel für ein Digitalbild 66, wie es mittels der Vorrichtung 20 sowie einer einen CCD-Flächensensor umfassenden Sensorik 30 beim automatischen Überwachen einer jeweiligen Anlage 10, insbesondere einer Anlage 10 der Luft- und Klimatechnik, aufgenommen worden sein könnte. Das Digitalbild 66 ist eine digitale Entsprechung einer mittels der Sensorik 30 jeweils aufgenommenen Szenerie. Das Digitalbild 66 befindet sich im Speicher 62 der Vorrichtung 20 (wurde als Ergebnis einer grundsätzlich an sich bekannten Verarbeitung mittels der Verarbeitungseinheit 68 in den Speicher 62 geladen). Die aufgenommene Szenerie ist die Oberfläche einer in der Auffangwanne 12 befindlichen Flüssigkeit oder eines Flüssigkeitsgemisches. Die Flüssigkeit ist Wasser, insbesondere Regenwasser, und das Flüssigkeitsgemisch umfasst Wasser, insbesondere Regenwasser.

Dem in Figur 7 gezeigten Digitalbild 66 liegt die Annahme zugrunde, dass aus der überwachten Anlage 10 aufgrund einer Fehlersituation Öl ausgetreten ist, sich in der Auffangwanne 12 unter der Anlage 10 Wasser, zum Beispiel Wasser in Form von Regenwasser, befindet und dass das ausgetretene Öl in Form eines dünnen Films (Ölfilm 60) auf der Wasseroberfläche schwimmt. Ein Betrachter erkennt eine solche dünne (Öl-)Schicht auf einer Wasseroberfläche oftmals als einen aufgrund von Lichtinterferenz entstehenden Farbverlauf (Regenbogenfarben). Der Farbverlauf umfasst die Spektralfarben des mit dem Auge sichtbaren Bereichs des Sonnenspektrums, nämlich violett (V), dunkelblau (D), hellblau (H), grün (G), gelb (Y), orange (O) und rot (R).

Aufgrund eines solchen Farbverlaufs resultierende Farbflächen und deren Farben lassen sich mittels der Vorrichtung 20 sowie mittels eines mittels der Vorrichtung 20 aufgenommenen Digitalbilds 66 erfassen und die Darstellung in Figur 7 zeigt schematisch vereinfacht einen Ausschnitt aus einem solchen Digitalbild 66. Ein Digitalbild 66 eines Ölfilms 60 auf einer Wasseroberfläche oder eines Teils eines solchen Ölfilms 60 unterscheidet sich von einem Digitalbild 66 einer Wasseroberfläche ohne einen Ölfilm 60 durch die Farbigkeit oder zumindest durch den Umfang der Farbigkeit. Entsprechend resultieren bei einer Situation ohne Ölfilm 60 andere Emissionsspektren 44, 46 (Histogramme) als im Falle einer Situation mit Ölfilm 60.

Der hier vorgeschlagene Ansatz macht sich zunutze, dass bei einer Situation ohne Ölfilm 60 bei einer ersten Beleuchtung 40 und einer anschließenden, spektral unterschiedlichen zweiten Beleuchtung 42 Emissionsspektren 44, 46 mit Spektrallinienpaaren resultieren, deren Amplitudenverhältnisse gleich oder zumindest im Wesentlichen gleich sind. Im Gegensatz dazu resultieren bei einer Situation mit Ölfilm 60 bei einer ersten Beleuchtung 40 und einer anschließenden, spektral unterschiedlichen zweiten Beleuchtung 42 Emissionsspektren 44, 46 mit Spektrallinienpaaren, bei denen die Amplitudenverhältnisse ungleich und auch nicht im Wesentlichen gleich sind.

Die Emissionsspektren 44, 46 und davon umfasste Spektrallinien 48 sind jeweils das Ergebnis einer Verarbeitung (Auswertung 50) eines Digitalbilds 66 und die im Falle einer Situation mit Ölfilm 60 resultierenden Emissionsspektren 44, 46 sind jeweils das Ergebnis einer Verarbeitung (Auswertung 50) eines Digitalbilds 66 wie beispielhaft in Figur 7 gezeigt. Das jeweilige Digitalbild 66 ist in der weiter oben verwendeten Terminologie eine Messung 44, 46 oder das Ergebnis einer Messung 44, 46. Die Darstellung in Figur 3 zeigt resultierende Spektrallinien 48 ohne Verschmutzung und die Darstellung in Figur 5 zeigt resultierende Spektrallinien 48 im Falle eines Ölfilms 60.

Optional sind die Vorrichtung 20 und/oder eine Ausführung des Überwachungsverfahrens mittels der Vorrichtung 20 automatisch aktivierbar und deaktivierbar. Zu diesem Zweck ist in der Auffangwanne 12 ein optionaler Feuchtigkeitssensor (nicht gezeigt) vorgesehen. Die Vorrichtung bzw. die Ausführung des Überwachungsverfahrens wird aktiviert, wenn der Feuchtigkeitssensor ein Feuchtigkeit in der Auffangwanne 12 anzeigendes Signal, insbesondere ein Wasser in der Auffangwanne 12 anzeigendes Signal, liefert sowie bei einem komplementären Signal deaktiviert.

Nochmals weiter optional sind die Vorrichtung 20 oder zumindest die von der Vorrichtung 20 umfasste Sensorik 30 beweglich, zum Beispiel in ein Schutzgehäuse hinein und aus dem Schutzgehäuse heraus, wobei eine Bewegung automatisch in Abhängigkeit von dem vom Feuchtigkeitssensor erhältlichen Signal gesteuert wird. Bei einem Feuchtigkeit, insbesondere Wasser, in der Auffangwanne 12 anzeigenden Signal wird die Vorrichtung 20 oder zumindest deren Sensorik 30 in eine Messposition bewegt (zum Beispiel verschwenkt), nämlich eine Position, in welcher die oben beschriebenen Messungen 44, 46 möglich sind, und bei einem komplementären Signal wird die Vorrichtung 20 oder zumindest deren Sensorik 30 in eine Schutzposition bewegt, in welcher diese gegen Beschädigungen oder Verschmutzung geschützt ist.

Zusammenfassend lässt sich bezüglich der hier vorgeschlagenen Neuerung Folgendes festhalten: Angegeben werden ein Verfahren (Überwachungsverfahren) und eine nach dem Verfahren arbeitende Vorrichtung (Überwachungsvorrichtung) 20 zur automatischen Überwachung einer Anlage 10 der Luft- und Klimatechnik mittels einer Vorrichtung 20. Eine solche Anlage 10 ist zur Vermeidung eines Austritts eines wasser- und/oder umweltgefährdenden Fluids regelmäßig in einer sogenannten Auffangwanne 12 platziert. Diese Möglichkeit besteht auch für Anlagen 10 wie zum Beispiel Notstromaggregate oder Trafostationen. Zusammenfassend werden alle in Betracht kommenden Anlagen 10 kurz als Anlagen 10 bezeichnet. Die jeweilige Anlage 10 wird automatisch mittels der hier vorgeschlagenen Vorrichtung 20 und gemäß dem hier vorgeschlagenen Verfahren überwacht. Die Vorrichtung 20 umfasst eine optische Sensorik 30 und eine Lichtquelle 32. Mittels der Lichtquelle 32 werden eine erste Beleuchtung 40 und eine im Vergleich zu der ersten Beleuchtung 40 spektral unterschiedliche zweite Beleuchtung 42 für eine mittels der Sensorik 30 aufgenommene erste und zweite Messung 44, 46 erzeugt. Aufgrund der ersten und zweiten Messung 44, 46 resultiert zum Beispiel ein erstes und zweites Emissionsspektrum 44, 46, jeweils mit Spektrallinien 48. In den beiden Emissionsspektren 44, 46 wird jeweils ein Paar Spektrallinien 48 - Spektrallinienpaar - bei gleichen Wellenlängen betrachtet. Im Rahmen einer Auswertung 50 der Spektrallinienpaare wird ein Verhältnis der Amplituden der beiden Spektrallinienpaare gebildet. Im Rahmen einer Fallunterscheidung 52 werden die ermittelten Amplitudenverhältnisse miteinander verglichen und in Abhängigkeit vom Ergebnis des Vergleichs erfolgt ggf. eine Fehlerbehandlung 54. Allgemeiner formuliert resultieren aufgrund der ersten Messung 44 ein erstes Datum und aufgrund der zweiten Messung 46 ein zweites Datum und im Rahmen einer Auswertung 50 wird ein Verhältnis des ersten und zweiten Datums jeder Messung 44, 46 gebildet. Im Rahmen einer Fallunterscheidung 52 werden die ermittelten Verhältnisse miteinander verglichen und in Abhängigkeit vom Ergebnis des Vergleichs erfolgt ggf. eine Fehlerbehandlung 54.

Die Vorrichtung 20 arbeitet nach dem Verfahren. Im Fehlerfall ist mittels der Vorrichtung 20 ein Fehlersignal 22 generierbar und wird im Rahmen des Verfahrens ein Fehlersignal 22 generiert. Die vorgeschlagene Neuerung ist also kurz gefasst unter anderem ein Verfahren und eine Vorrichtung zur Überwachung einer Anlage 10 der Luft- und Klimatechnik, wobei aufgrund einer ersten Beleuchtung 40 und einer späteren, im Vergleich zu der ersten Beleuchtung 40 spektral unterschiedlichen zweiten Beleuchtung 42 mittels einer Sensorik 30 eine erste und eine zweite Messung 44, 46 einer Szenerie unterhalb der Anlage 10 erfolgt, wobei aufgrund der Messungen 44, 46 zwei Emissionsspektren 44, 46 mit Spektrallinien 48 resultieren, wobei in den Emissionsspektren 44, 46 jeweils ein Spektrallinienpaar bei gleichen Wellenlängen betrachtet und deren Amplitudenverhältnis ermittelt wird und wobei die Amplitudenverhältnisse miteinander verglichen werden und in Abhängigkeit vom Ergebnis des Vergleichs ggf. eine Fehlerbehandlung 54 erfolgt.

### Bezugszeichenliste

- 10: Anlage der Luft- und Klimatechnik, Klimagerät
- 12: Auffangwanne
- 14: Auffang- und Rückhaltesystem, Rückhaltesystem
- 16,18: (frei)
- 20: Vorrichtung, Überwachungsvorrichtung
- 22: Fehlersignal
- 24: Ventil
- 26: Ablauf
- 28: (frei)
- 30: Sensorik
- 32: Lichtquelle, Strahlquelle
- 34: Gehäuse (der Vorrichtung/Überwachungsvorrichtung)
- 36, 38: (frei)
- 40: erste Beleuchtung
- 42: zweite Beleuchtung
- 44: erste Messung, erstes Emissionsspektrum
- 46: zweite Messung, zweites Emissionsspektrum
- 48: Spektrallinie
- 50: Auswertung
- 52: Fallunterscheidung
- 54: Fehlerbehandlung
- 56: Abbruchkriterium
- 58: (frei)
- 60: Ölfilm
- 62: Speicher
- 64: Optik
- 66: Digitalbild
- 68: Verarbeitungseinheit
- 70: Steuerungsprogramm

## Patentansprüche

1. Verfahren zur Überwachung einer Anlage (10) der Luft- und Klimatechnik mittels einer Vorrichtung (20),
wobei die Anlage (10) eine Auffangwanne (12) umfasst oder der Anlage (10) eine Auffangwanne (12) zugeordnet ist,
wobei die Vorrichtung (20) eine jeweils in die Auffangwanne (12) gerichtete optische Sensorik (30) und Lichtquelle (32) umfasst,
wobei mittels der Lichtquelle (32) eine erste Beleuchtung (40) und eine im Vergleich zu der ersten Beleuchtung spektral unterschiedliche zweite Beleuchtung (42) für eine mittels des Sensorik (30) aufgenommene erste und zweite Messung (44, 46) erzeugt werden,
wobei aufgrund der ersten und zweiten Messung (44, 46) jeweils ein erstes Datum und ein zweites Datum resultieren,
wobei im Rahmen einer Auswertung (50) ein Verhältnis des ersten und zweiten Datums jeder Messung (44, 46) gebildet wird,
wobei im Rahmen einer Fallunterscheidung (52) die ermittelten Verhältnisse miteinander verglichen werden und
wobei in Abhängigkeit vom Ergebnis des Vergleichs eine Fehlerbehandlung (54) erfolgt.

2. Verfahren nach Anspruch 1,
wobei aufgrund der ersten und zweiten Beleuchtung (40, 42) ein mittels der ersten und zweiten Messung (44, 46) aufgenommenes erstes und zweites Emissionsspektrum (44, 46) mit Spektrallinien (48) resultiert,
wobei in den beiden Emissionsspektren (44, 46) jeweils die Spektrallinien (48) eines Spektrallinienpaars bei gleichen Wellenlängen betrachtet werden und das aufgrund jeder Messung (44, 46) resultierende erste Datum und zweite Datum jeweils die Amplitude der betrachteten Spektrallinien (48) ist,
wobei im Rahmen der Auswertung (50) ein Verhältnis der Amplituden der beiden Spektrallinienpaare gebildet wird,
wobei im Rahmen einer Fallunterscheidung (52) die ermittelten Amplitudenverhältnisse miteinander verglichen werden und
wobei in Abhängigkeit vom Ergebnis des Vergleichs eine Fehlerbehandlung (54) erfolgt.

3. Vorrichtung (20) mit Mitteln (30, 32, 62, 64, 68) zur Ausführung eines Verfahrens nach einem der Ansprüche 1 oder 2.

4. Vorrichtung nach Anspruch 3 und
mit einer Sensorik (30) sowie einer Lichtquelle (32) als Mittel (30, 32, 62, 64, 68) zur Ausführung eines Verfahrens nach einem der Ansprüche 1 oder 2,
wobei mittels der Lichtquelle (32) beim Betrieb der Vorrichtung (20) die erste Beleuchtung (40) und die im Vergleich zu der ersten Beleuchtung (40) spektral unterschiedliche zweite Beleuchtung (42) für eine mittels der Sensorik (30) aufnehmbare erste und zweite Messung (44, 46) erzeugt wird und
wobei mittels der Sensorik (30) beim Betrieb der Vorrichtung (20) die erste und die zweite Messung (44, 46) aufgenommen wird.

5. Vorrichtung nach Anspruch 4,
wobei die Lichtquelle (32) der Sensorik (30) als Ringlicht zugeordnet ist.

6. Vorrichtung (20) nach Anspruch 3, 4 oder 5 und
mit einer Sensorik (30) als Mittel (30, 32, 62, 64, 68) zur Ausführung eines Verfahrens nach einem der Ansprüche 1 oder 2,
wobei die Sensorik (30) einen ersten und einen zweiten Sensor jeweils in Form einer Photodiode umfasst und
wobei ein Erfassungsbereich des ersten Sensors einem Wellenlängenbereich der ersten Beleuchtung (40) entspricht oder den Wellenlängenbereich der ersten Beleuchtung (40) zumindest umfasst und ein Erfassungsbereich des zweiten Sensors einem Wellenlängenbereich der zweiten Beleuchtung (42) entspricht oder den Wellenlängenbereich der zweiten Beleuchtung (42) zumindest umfasst.

7. Vorrichtung (20) nach Anspruch 3, 4 oder 5
und mit einer Sensorik (30) als Mittel (30, 32, 62, 64, 68) zur Ausführung eines Verfahrens nach einem der Ansprüche 1 oder 2,
wobei die Sensorik (30) zumindest einen Sensor in Form eines CCD-Flächensensors oder eines CCD-Zeilensensors umfasst und
wobei ein Erfassungsbereich des Sensors einen Wellenlängenbereich der ersten und zweiten Beleuchtung (40, 42) umfasst oder jeweils ein solcher Sensor den Wellenlängenbereich der ersten bzw. zweiten Beleuchtung (40, 42) umfasst.

8. Steuerungsprogramm (70) mit einer Implementation eines Verfahrens nach einem der Ansprüche 1 oder 2.

9. Vorrichtung (20) nach einem der Ansprüche 3 bis 7 und mit einer Implementation eines Verfahrens nach einem der vorangehenden Verfahrensansprüche oder mit Mitteln (30, 32, 62, 64, 68, 70) zur Ausführung eines Verfahrens nach einem der vorangehenden Verfahrensansprüche oder mit einem Speicher (62), in den ein Steuerungsprogramm (70) nach Anspruch 8 geladen ist.

10. Verwendung einer Vorrichtung (20) nach einem der vorangehenden Vorrichtungsansprüche oder eines Verfahrens nach einem der vorangehenden Verfahrensansprüche zur Überwachung einer Anlage (10) der Luft- und Klimatechnik in Bezug auf einen Austritt eines wasser- und/oder umweltgefährdenden Fluids aus der Anlage (10).
